# FASCICULE DE BREVET EUROPEEN

(11) **EP 2 068 896 B1**
(45) Date de publication et mention de la délivrance du brevet: **08.03.2017**
(21) Numéro de dépôt: 07823881.3
(22) Date de dépôt: 28.09.2007
(51) Int. Cl.: A61K 35/08, A61K 9/00, A61P 11/02, A61K 33/00, A61K 33/06, A61K 33/14

(54) **SOLUTIONS IONIQUES HYPERTONIQUES A BASE D'EAU DE MER NON DILUEE, LEUR UTILISATION EN TANT QUE COMPOSITIONS PHARMACEUTIQUES OU MEDICAMENTS ET POUR LA PREPARATION DE MEDICAMENTS DESTINES AU TRAITEMENT DE LA CONGESTION NASALE**
IONISCHE HYPERTONE LÖSUNGEN AUF BASIS VON UNVERDÜNNTEM MEERWASSER, IHRE VERWENDUNG ALS PHARMAZEUTISCHE MITTEL ODER MEDIKAMENT UND ZUR HERSTELLUNG EINES MEDIKAMENTS ZUR BEHANDLUNG VON NASENVERSTOPFUNG
IONIC HYPERTONIC SOLUTIONS BASED ON UNDILUTED SEAWATER, THEIR USE AS PHARMACEUTICAL COMPOSITIONS OR MEDICATION AND FOR PREPARING MEDICATION INTENDED FOR TREATING NASAL CONGESTION

(30) Priorité: 29.09.2006 FR 0608595
(43) Date de publication de la demande: 17.06.2009
(73) Titulaire: Laboratoire de la Mer, 35400 Saint-Malo (FR)
(72) Inventeur: YVIN, Jean-Claude, 35400 Saint Malo (FR); ANDRO, François, 35630 Hede (FR)
(74) Mandataire: Le Guen-Maillet
(86) Numéro de dépôt international: PCT/FR2007/052047
(87) Numéro de publication internationale: WO 2008/037938

(56) Documents cités:
- WO-A-2006/056801
- CA-C- 1 313 142
- FR-A1- 2 299 041
- US-B1- 7 097 852
- MICHEL O: "Nasal irrigation in case of rhinosinusitis" LARYNGO- RHINO- OTOLOGIE 2006 GERMANY, vol. 85, no. 6, 2006, pages 448-458, XP009079014 ISSN: 0935-8943
- TRAISSAC L ET AL: "LE LAVAGE DE NEZ AVEC PHYSIOMER... 10 ANS APRES: 1988-1998" REVUE DE LARYNCOLOGIE, D'OTOLOGIE ET DE RHINOLOGIE, DOIN, PARIS, FR, vol. 120, no. 2, 1999, pages 133-135, XP000882069 ISSN: 0035-1334

## Description

L'invention a pour objet des solutions ioniques hypertoniques à base d'eau de mer non diluée et des compositions pharmaceutiques ou médicaments les comprenant. Elle vise également l'utilisation des solutions en tant que compositions pharmaceutiques ou médicaments, leur utilisation pour la préparation de médicaments destinés au traitement de la congestion nasale ainsi que leur procédé de préparation.

La Société Demanderesse a déjà décrit l'application des solutions ioniques à base d'eau de mer hypotoniques dans la prévention, l'hygiène et le traitement des voies respiratoires, buccales, de la peau et des muqueuses gynécologiques, notamment dans les brevets EP1093374 et EP1196185.

Par ailleurs, la Société Demanderesse a développé des solutions ioniques aqueuses du genre en question, dont la composition ionique quantitative, le pH et l'osmolalité ont été adaptées pour une utilisation pour le traitement de l'hygiène de l'oeil et de ses annexes. Ces solutions sont décrites notamment dans la demande de brevet FR2803205.

D'autres équipes de chercheurs se sont intéressées à l'utilisation de solutions ioniques hypertoniques, de synthèse, le plus souvent tamponnées, comme complément aux traitements classiques des rhinites allergiques (Garavello W. et al., « Hypersaline nasal irrigation in children with symptomatic seasonal allergic rhinitis : a randomized study » Pediatr. Allergy Immunol 2003 ; 14(2) : 140-3), rhinites chroniques, sinusites aiguës et chroniques (Rabago D. et al. « Efficacy of daily hypertonic saline nasal irrigation among patients with sinusitis : a randomized controlled trial » The Journal of family practice, December 2002, vol.51, n°12, p. 1049-1055; Andrew R. Talbot et al. "Mucociliary Clearance and Buffered hypertonic Saline Solution", Laryngoscope, 107:500-503, 1997). Les résultats obtenus n'ont pas toujours été probants et les solutions salines hypertoniques se sont avérées mal tolérées (Adam P. « A clinical trial of hypertonic saline nasal spray in subjects with the common cold or rhinosinusitis » Arch Fam Med, 7(1) :39-43, 1998 Jan-Feb.).

Dans le brevet CA1313142, l'utilisation d'une eau de mer dont la composition est caractérisée par sa région de prélèvement, au large du Canada, est décrite comme désinfectant et décongestionnant des muqueuses notamment nasales. Cependant, l'utilisation de cette solution engendre des phénomènes d'intolérance pour la muqueuse nasale et perturbe la physiologie de ladite muqueuse. Pour remédier à ces inconvénients, il a été proposé dans FR 2 769 503 d'utiliser une solution hypertonique d'eau de mer diluée avec 30% d'eau distillée.

La congestion nasale peut être le fait d'une sinusite, rhinite chronique, d'origine allergique notamment. Elle accompagne souvent les asthmes allergiques. Elle se manifeste par des symptômes tels que des picotements dans la cavité nasale, des éternuements, une rhinorrhée plus ou moins importante et la sensation de nez bouché.

Des traitements de fond sont administrés aux patients souffrant de tels symptômes, notamment des traitements à base de corticostéroïdes. Ces corticostéroïdes oraux ou administrés par voie nasale ont pour fonction d'agir sur la réduction du gonflement des muqueuses et sur les sécrétions en diminuant directement ou indirectement la concentration des médiateurs de l'inflammation (histamine et prostaglandine par exemple) libérés dans le nez. Cependant, à long terme, de tels traitements provoquent d'importants effets secondaires et/ou les patients ne supportent plus ces traitements.

Il existe donc un réel besoin en une composition pharmaceutique ou médicament bien toléré pour des traitements à long terme, ne provoquant pas d'effet secondaire et qui permette la diminution voire l'arrêt de tout traitement classique utilisé pour la congestion nasale, notamment de type corticostéroïde, en particulier corticostéroïdes pour administration par voie nasale.

A la suite de recherches longues et approfondies, la Société Demanderesse a trouvé que l'utilisation de solutions ioniques hypertoniques à base d'eau de mer permettait de répondre à ces exigences.

Ainsi, la présente invention porte sur les solutions ioniques hypertoniques mises au point par la Société Demanderesse et qui sont caractérisées par le fait qu'elles sont obtenues à partir d'eau de mer, qu'elles sont non diluées et qu'elles présentent :
- une osmolalité comprise entre 500 à 700 mOsm/kg, de préférence entre 550 et 700 mOsm/kg, et plus préférentiellement encore entre 600 et 650 mOsm/kg,
- un pH compris entre 7,5 et 9, une composition du point de vue ionique qui est qualitativement celle de l'eau de mer et qui est telle que le rapport Na⁺/Mg⁺⁺ soit inférieur à 6, de préférence compris entre 3,5 et 5, et plus préférentiellement encore compris entre 4,5 et 5.

Du point de vue qualitatif, la composition ionique des solutions mises en oeuvre conformément à l'invention est celle de l'eau de mer.

A titre illustratif, on indique dans le tableau ci-après la composition de l'eau de mer telle qu'elle figure à la page F 163 de l'ouvrage Handbook of Chemistry and Physics 63ème édition, 1982-1983, CRC PRESS.

**TABLEAU**

| Elément | Quantité (ppm) |
|---|---|
| Cl | 18,980 |
| Na | 10,561 |
| Mg | 1,272 |
| S | 884 |
| Ca | 400 |
| K | 380 |
| Br | 65 |
| C (inorganique) | 28 |
| Sr | 13 |
| (SiO₂) | 0,01-7,0 |
| B | 4,6 |
| Si | 0,02-4,0 |
| C (organique) | 1,2-3,0 |
| Al | 0,16-1,9 |
| F | 1,4 |
| N (en tant que nitrate) | 0,001-0,7 |
| N (en tant qu'azote organique) | 0,03-0,2 |
| Rb | 0,2 |
| Li | 0,1 |
| P (en tant que phosphate) | >0,001-0,10 |
| Ba | 0, 05 |
| I | 0, 05 |
| N (en tant que nitrite) | 0,0001-0,05 |
| N (en tant qu'ammoniaque) | >0,005-0,05 |
| As (en tant qu'arsenic) | 0,003-0,024 |
| Fe | 0,002-0,02 |
| P (en tant que phosphore organique) | 0,016 |
| Zn | 0,005-0,014 |
| Cu | 0,001-0,09 |
| Mn | 0,001-0,01 |
| Pb | 0,004-0,005 |
| Se | 0, 004 |
| Sn | 0, 003 |
| Cs | 0,002 (approximativement) |
| U | 0,00015-0,0016 |
| Mo | 0,0003-0,002 |
| Ga | 0,0005 |
| Ni | 0,0001-0,0005 |
| Th | <0,0005 |
| Ce | 0,0004 |
| V | 0,0003 |
| La | 0,0003 |
| Y | 0,0003 |
| Hg | 0,00003 |
| Ag | 0,00015-0,0003 |
| Bi | 0,0002 |
| Co | 0,0001 |
| Sc | 0,00004 |
| Au | 0,000004-0,000008 |
| Fe (en solution vraie) | <10⁻⁹ |
| Ra | 2.10⁻¹¹-3.10⁻¹⁰ |
| Ge | Présent |
| Ti | Présent |
| W | Présent |
| Cd | Présent dans des organismes marins |
| Cr | Présent dans des organismes marins |
| Tl | Présent dans des organismes marins |
| Sb | Présent dans des organismes marins |
| Zr | Présent dans des organismes marins |
| Pt | Présent dans des organismes marins |

A la même page du même ouvrage il est précisé que le pH de l'eau de mer est de 8-9.

On sait par ailleurs (IFREMER, Département Environnement Littoral et Gestion du milieu marin) que l'osmolalité de l'eau de mer est supérieure ou égale à 1000 mOsm/kg.

On sait également que dans l'eau de mer brute, le rapport Na/Mg est supérieur à 8.

Selon un mode de réalisation avantageux, les solutions ioniques hypertoniques conformes à l'invention présentent une composition du point de vue ionique qui est qualitativement celle de l'eau de mer, à l'exception en particulier du point de vue quantitatif, des concentrations en Na⁺, K⁺, Mg⁺⁺, Ca⁺⁺ et Cl⁻, lesdites concentrations étant approximativement :
- pour Na⁺, de 5000 à 19000, de préférence de 5500 à 8000, et plus préférentiellement encore de 6000 à 6500 mg/l
- pour K⁺, de 50 à 650 de préférence de 100 à 250 mg/l
- pour Mg⁺⁺, de 1000 à 4000, de préférence de 1000 à 2000, et plus préférentiellement encore de 1100 à 1500 mg/l
- pour Ca⁺⁺, de 300 à 500, de préférence de 350 à 450 mg/l
- pour Cl⁻, de 8000 à 33000, de préférence de 9000 à 15000, et plus préférentiellement encore de 10000 à 13000 mg/l.

Dans le cas de l'eau de mer, les valeurs correspondantes sont matérialisées par les fourchettes reflétant les résultats de 134 mesures effectuées sur de l'eau de mer prélevée au large de Saint-Malo d'août 1998 à juillet 1999, à savoir:

| | |
|---|---|
| pH | : 7,70 à 8,30 |
| osmolalité | : >1000 mOsm/kg |
| [Na⁺] | : 10500-11500 mg/l |
| [K⁺] | : 365-420 mg/l |
| [Mg⁺⁺] | : 1200-1450 mg/l |
| [Ca⁺⁺] | : 380-435 mg/l |
| [Cl⁻] | : 18900-20500 mg/l |
| Rapport Na/Mg | : >8. |

Outre les éléments mentionnés ci-dessus, la solution hypertonique selon l'invention contient également du brome (Br), de l'aluminium (Al), du fluor (F), de l'iode (I), du fer (Fe), du zinc (Zn), du cuivre (Cu), du manganèse (Mn), du sélénium (Se).

De façon générale, dans la solution ionique hyperosmotique à base d'eau de mer, la composition en les éléments autres que le sodium, le potassium, les chlorures, le calcium et le magnésium est qualitativement identique à celle de l'eau de mer.

L'invention vise également en tant que composition pharmaceutique ou médicament les solutions ioniques hypertoniques conformes à l'invention.

Elle a donc pour objet les médicaments ou compositions pharmaceutiques caractérisés par le fait qu'ils sont à base des solutions ioniques hypertoniques conformes à l'invention.

L'invention vise également des médicaments ou compositions pharmaceutiques qui sont à base d'une solution ionique hypertonique à base d'eau de mer, non diluée et présentant
- une osmolalité comprise entre 500 et 700 mOsm/kg, de préférence entre 550 et 700 mOsm/kg, et plus préférentiellement encore entre 600 et 650 mOsm/kg,
- un pH compris entre 7,5 et 9,
- une composition du point de vue ionique qui est qualitativement celle de l'eau de mer, qui est telle que le rapport Na⁺/Mg⁺⁺ soit inférieur à 6, de préférence compris entre 3,5 et 5, et plus préférentiellement encore compris entre 4,5 et 5.
Dans ces médicaments, les concentrations en Na⁺, K⁺, Mg⁺⁺, Ca⁺⁺ et Cl⁻ sont les suivantes :
- pour Na⁺, de 5000 à 19000, de préférence de 5500 à 8000, et plus préférentiellement encore de 6000 à 6500 mg/l
- pour K⁺, de 50 à 650, de préférence de 100 à 250 mg/l
- pour Mg⁺⁺, de 1000 à 4000, de préférence de 1000 à 2000, et plus préférentiellement encore de 1100 à 1500 mg/l
- pour Ca⁺⁺, de 300 à 1200, de préférence de 350 à 450 mg/l
- pour Cl⁻, de 8000 à 33000, de préférence de 9000 à 15000 et plus préférentiellement encore de 10000 à 13000 mg/l, la composition qualitative et quantitative en autres éléments étant celle de l'eau de mer.

Selon un mode de réalisation particulier, la composition pharmaceutique ou le médicament conforme à l'invention contient également du brome (Br), de l'aluminium (Al), du fluor (F), de l'iode (I), du fer (Fe), du zinc (Zn), du cuivre (Cu), du manganèse (Mn), du sélénium (Se).

Selon un autre mode de réalisation du médicament conforme à l'invention, dans la solution ionique hyperosmotique à base d'eau de mer, la composition en les éléments autres que le sodium, le potassium, les chlorures, le calcium et le magnésium est qualitativement identique à celle de l'eau de mer.

Selon un mode de réalisation avantageux, la solution pharmaceutique ou le médicament conforme à l'invention se présente sous la forme d'une solution pour pulvérisation nasale.

Les médicaments conformes à l'invention sont dépourvus de tout agent conservateur ou stabilisant. Ce dernier avantage est d'une grande importance.

En effet, les agents conservateurs présents dans la plupart des solutions ioniques de synthèse, provoquent à plus ou moins long terme des effets secondaires.

L'invention a également pour objet l'utilisation des solutions ioniques hypertoniques à base d'eau de mer conformes à l'invention pour la préparation d'un médicament destiné au traitement curatif ou prophylactique de la congestion nasale.

L'invention est particulièrement adaptée au traitement de patients traités par corticostéroïdes ou sensibles aux corticostéroïdes, notamment aux corticostéroïdes pour administration par voie nasale.

Ainsi, l'invention porte sur l'utilisation des solutions ioniques hypertoniques de l'invention pour la préparation de médicaments destinés au traitement préventif ou prophylactique de la congestion nasale chez des patients traités par corticostéroïdes.

L'utilisation conforme à l'invention peut se faire ou bien en traitement supplétif des traitements à base de corticostéroïdes en vue de diminuer les doses journalières de corticostéroïdes et /ou la durée du traitement, ou bien en traitement substitutif aux corticostéroïdes, au moins pendant une période prolongée.

Les solutions hypertoniques de l'invention, représentent une solution efficace et alternative aux corticostéroïdes locaux. Elles peuvent permettre l'interruption prolongée de la prise de corticostéroïdes.

La solution conforme à l'invention peut être administrée dans les cavités nasales par instillation, nébulisation ou pulvérisation. De façon préférée, elle est administrée par pulvérisation.

A titre d'exemple, on signale que l'on peut procéder à 6 applications par jour, notamment pour un traitement curatif. Pour un traitement prophylactique, 3 applications par jour peuvent s'avérer suffisantes.

De façon tout à fait surprenante, il s'est avéré que les solutions hypertoniques conformes à l'invention étaient très bien tolérées par les patients qui ne se plaignaient ni de brûlures ni de picotements lors de l'administration notamment par pulvérisation. En outre, la tolérance au produit s'accroît au cours du temps.

Un autre objet de l'invention concerne le procédé de préparation de la solution ionique conforme à l'invention, selon lequel l'eau de mer est traitée par électrodialyse. Plus particulièrement, selon le procédé, successivement :
- on prélève, en tant que matière première, de l'eau de mer d'une teneur en sels supérieure à 32g/l, avantageusement par une profondeur de 5 à 10 mètres dans une zone à forts mouvements de courant,
- on clarifie, filtre et analyse cette eau,
- si l'osmolalité de l'eau de mer clarifiée et filtrée est supérieure à l'osmolalité souhaitée, on la désode par électrodialyse jusqu'à l'obtention de l'osmolalité souhaitée qui est supérieure à 500 mOsm/kg,
- si l'osmolalité de l'eau de mer décantée est inférieure à l'osmolalité souhaitée, on la concentre jusqu'à l'obtention de l'osmolalité souhaitée qui est inférieure à 700 mOsm/kg,
- on ajuste les concentrations ioniques par électrodialyse sélective,
- on filtre et on stocke le produit dans des conditions stériles.

### EXEMPLES

### EXEMPLE 1

On prépare une solution ionique hypertonique conforme à l'invention, à partir d'eau de mer puisée au large de Saint-Malo et prélevée à une profondeur de 5 à 10 mètres dans une zone à forts mouvements de courant; cette eau est caractérisée par une teneur en sels supérieure à 32 g/1; elle est naturellement riche en calcium, magnésium et autres oligo-éléments.

Cette eau est soumise à une électrodialyse sélective; dans un premier temps, on retire essentiellement le chlorure de sodium pour atteindre l'osmolalité souhaitée, puis on ajuste les concentrations ioniques; le pH recherché est obtenu de préférence en échangeant les ions Na⁺ contre des protons.

L'électrodialyse sélective est effectuée avec un appareil du type EUR 6B commercialisé par la Société EURODIA Industrie SA sous la marque EURODIA. Les différentes étapes de l'électrodialyse sélective correspondant à l'ajustement à chacun des différents paramètres (pH, osmolalité, concentration en ions) sont effectuées de manière connue.

La solution obtenue est ensuite stérilisée et présente les caractéristiques suivantes :

| | |
|---|---|
| pH | :8 |
| osmolalité | :627 mOsm/l |
| Na | :6370 mg/l |
| K | :165 mg/l |
| Mg | :1315 mg/l |
| Ca | :360mg/l |
| Cl | :1200mg/l |
| Na/Mg | :4,84 |

### EXEMPLE 2

L'efficacité de la solution ionique hypertonique de l'exemple 1 a été vérifiée par une étude conduite sur une population randomisée de 238 patients volontaires de plus de 12 ans pendant 6 semaines consécutives.

Les patients souffraient de rhinite allergique ou de sinusite allergique depuis plus de deux ans et étaient sous corticostéroïde nasal depuis au minimum 4 semaines.

Les patients utilisaient l'un des corticostéroïdes suivants :
Budenoside par inhalation nasale
Motenasone par spray nasal
Fluticasone propionate par inhalation nasale
Beclomethasone dipropionate par inhalation nasale

Les patients ont été divisés en 3 groupes homogènes respectivement soumis aux traitements suivants :
groupe 1: poursuite du traitement corticostéroïde avec le corticostéroïde utilisé préalablement à l'étude,
groupe 2: pendant 2 semaines traitement à l'aide de la solution de l'invention à raison de 1 à 3 applications par jour en fonction du patient et du même corticostéroïde puis pendant 4 semaines traitement à l'aide de la solution de l'invention à raison de 3 fois par jour et application du même corticostéroïde uniquement en cas d'urgence
groupe 3: traitement à l'aide de la solution de l'invention à raison de 3 fois par jour pendant 6 semaines et application du même corticostéroïde uniquement en cas d'urgence.

Les patients des trois groupes ont été observés par un médecin le jour du commencement de l'étude (visite 1), deux semaines (visite 2) puis 6 semaines (visite 3) après le début de l'étude.

A chaque visite les critères suivants ont été évalués:
- efficacité (modification des symptômes et Indice NIS, l'indice NIS (Nasal Index Score) étant la somme cumulée des scores évalués de 0 à 3 pour chacun des symptômes suivants "rhinorrhée", "congestion", "picotement" et "éternuement" divisée par le score maximum de 12 ;
- nombre moyen d'applications de corticostéroïdes ;
- sécurité (nombre de complications et plaintes) ;
- évaluation de l'état de santé du patient par lui-même;
- état de santé évalué par le médecin ;
- sensation avant et après l'administration de la solution de l'invention (uniquement pour les groupes 2 et 3).

L'appréciation qualitative du picotement a été transposée sur une échelle numérique de la façon suivante:

| Picotement | Aucun | léger | modéré | Sévère |
|---|---|---|---|---|
| Echelle | 0 | 1 | 2 | 3 |

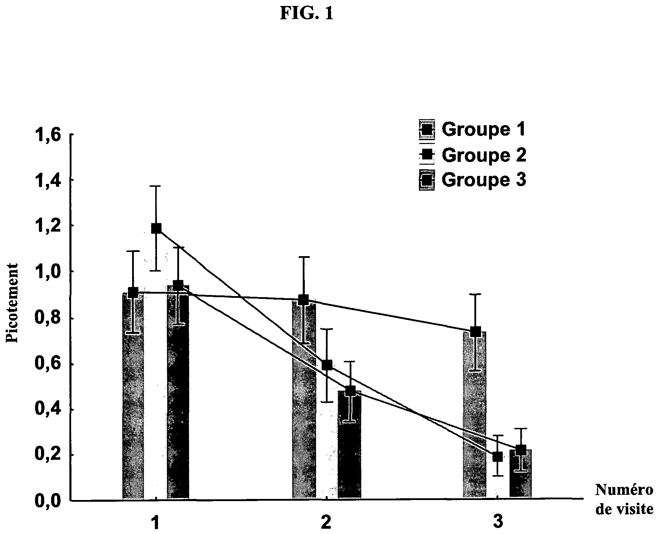

Les résultats obtenus sont donnés dans le tableau 1 ci-après et repris sous forme graphique sur la Figure 1 :

**Tableau 1**

| | Groupe 1 | Groupe 2 | Groupe 3 |
|---|---|---|---|
| Visite 1 | 0,9 | 1,2 | 0,9 |
| Visite 2 | 0,9 | 0,6 | 0,5 |
| Visite 3 | 0,7 | 0,2 | 0,2 |

Les patients des groupes 2 et 3 ont connu une diminution significative des picotements.

L'appréciation qualitative de l'éternuement a été transposée sur une échelle numérique de la façon suivante :

| Eternuement | Aucun | léger | modéré | Sévère |
|---|---|---|---|---|
| Echelle | 0 | 1 | 2 | 3 |

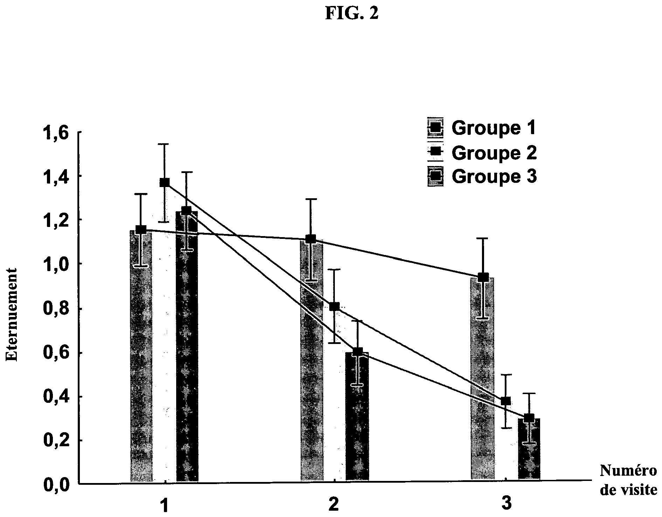

Les résultats obtenus sont donnés dans le tableau 2 ci-après et repris sous forme graphique sur la Figure 2 :

**Tableau 2**

| | Groupe 1 | Groupe 2 | Groupe 3 |
|---|---|---|---|
| Visite 1 | 1,2 | 1,4 | 1,2 |
| Visite 2 | 1,1 | 0,8 | 0,6 |
| Visite 3 | 0,9 | 0,4 | 0,3 |

Les patients des groupes 2 et 3 ont connu une diminution significative des éternuements.

L'appréciation qualitative de la rhinorrhée a été transposée sur une échelle numérique de la façon suivante :

| rhinorrhée | Aucune | légère | modérée | Sévère |
|---|---|---|---|---|
| Echelle | 0 | 1 | 2 | 3 |

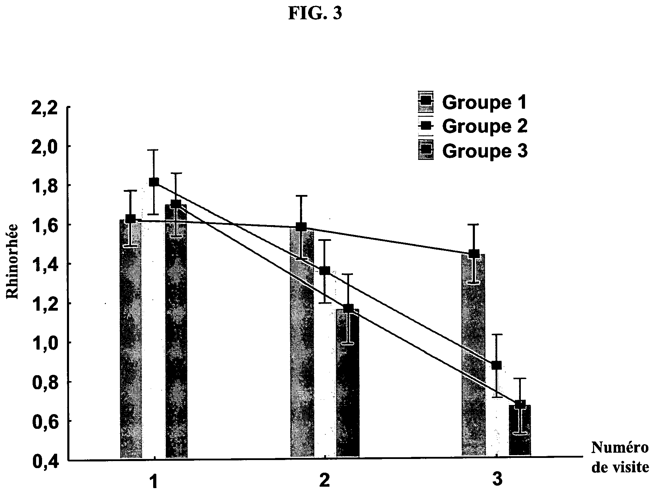

Les résultats obtenus sont donnés dans le tableau 3 ci-après et repris sous forme graphique sur la Figure 3 :

**Tableau 3**

| | Groupe 1 | Groupe 2 | Groupe 3 |
|---|---|---|---|
| Visite 1 | 1,6 | 1,8 | 1,7 |
| Visite 2 | 1,6 | 1,4 | 1,2 |
| Visite 3 | 1,4 | 0,9 | 0,7 |

Les patients des groupes 2 et 3 ont connu une diminution significative de la rhinorrhée alors que les patients du groupe 1, traités uniquement par corticostéroïdes n'ont connu qu'une très légère amélioration.

L'appréciation qualitative de la congestion a été transposée sur une échelle numérique de la façon suivante :

| Congestion | Aucune | légère | modérée | Sévère |
|---|---|---|---|---|
| Echelle | 0 | 1 | 2 | 3 |

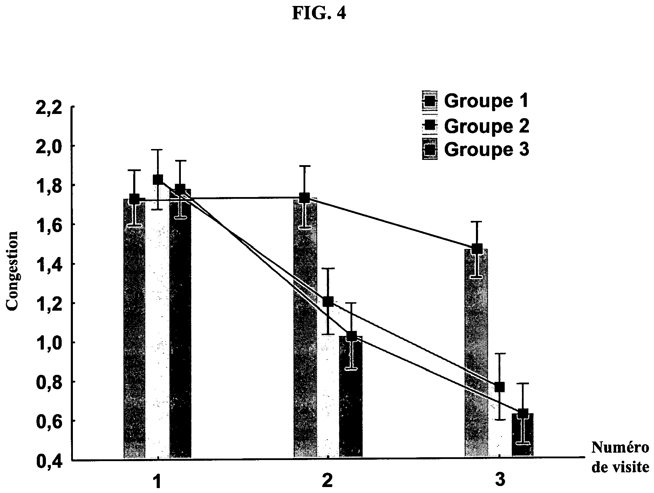

Les résultats obtenus sont donnés dans le tableau 4 ci-après et repris sous forme graphique sur la Figure 4 :

**Tableau 4**

| | Groupe 1 | Groupe 2 | Groupe 3 |
|---|---|---|---|
| Visite 1 | 1,7 | 1,8 | 1,8 |
| Visite 2 | 1,7 | 1,2 | 1,0 |
| Visite 3 | 1,5 | 0,8 | 0,6 |

Les patients des groupes 2 et 3 ont connu une diminution significative de la congestion, le meilleur effet étant constaté pour le groupe 3, alors que les patients du groupe 1, traités par corticostéroïdes n'ont connu qu'une très légère amélioration.

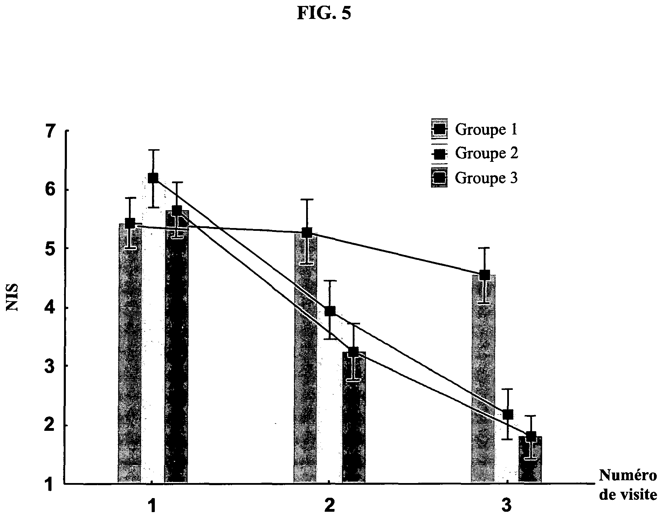

A l'aide de ces résultats on a calculé l'indice NIS. Les résultats sont présentés dans le tableau 5 ci-dessous et repris sous forme graphique sur la Figure 5 :

**Tableau 5**

| | Groupe 1 | Groupe 2 | Groupe 3 |
|---|---|---|---|
| Visite 1 | 5,42 | 6,19 | 5, 65 |
| Visite 2 | 5,27 | 3, 94 | 3,25 |
| Visite 3 | 4,54 | 2, 17 | 1,79 |

L'amélioration due au traitement par la solution ionique hypertonique selon l'invention (groupes 2 et 3) est très significative alors que l'amélioration est faible pour les patients traités par corticostéroïdes uniquement.

En ce qui concerne la prise de corticostéroïdes, la quantité nécessaire dans les deux premières semaines de l'étude a pu être réduite de moitié pour le groupe 2; puis il a été possible de supprimer les corticostéroïdes pendant les 4 semaines suivantes sauf pour quelques cas particuliers pour lesquels la recrudescence des symptômes était due à un pic de facteurs allergisants.

Pour le groupe 3, aucun patient n'a eu recours aux corticostéroïdes pendant les 4 dernières semaines de l'étude.

Au vu de ces résultats, il apparaît que les solutions hypertoniques selon l'invention, représentent une solution efficace pour le traitement curatif ou prophylactique de la congestion nasale, notamment chez des patients traités par ou sensibles aux corticostéroïdes, et constituent une alternative aux corticostéroïdes locaux. Elles permettent l'interruption prolongée de la prise de corticostéroïdes.

## Revendications

1. Solution ionique, hypertonique à base d'eau de mer, non diluée, présentant :
- une osmolalité comprise entre 500 et 700 mOsm/kg, de préférence entre 550 et 700 mOsm/kg, et plus préférentiellement encore entre 600 et 650 mOsm/kg,
- un pH compris entre 7,5 et 9,
- une composition du point de vue ionique qui est qualitativement celle de l'eau de mer et qui est telle que le rapport Na⁺/Mg⁺⁺ soit inférieur à 6, de préférence compris entre 3,5 et 5, et plus préférentiellement encore compris entre 4,5 et 5 et dont les concentrations en Na⁺, K⁺, Mg⁺⁺, Ca⁺⁺ et Cl⁻ sont les suivantes :
- pour Na⁺, de 5000 à 19000, de préférence de 5500 à 8000, et plus préférentiellement encore de 6000 à 6500 mg/l
- pour K⁺, de 50 à 650, de préférence de 100 à 250 mg/l
- pour Mg⁺⁺, de 1000 à 4000, de préférence de 1000 à 2000, et plus préférentiellement encore de 1100 à 1500 mg/l
- pour Ca⁺⁺, de 300 à 1200, de préférence de 350 à 450 mg/l
- pour Cl⁻, de 8000 à 33000, de préférence de 9000 à 15000, et plus préférentiellement encore de 10000 à 13000 mg/l.

2. Solution hypertonique selon la revendication 1, **caractérisée par le fait qu'**elle contient également du brome (Br), de l'aluminium (Al), du fluor (F), de l'iode (I), du fer (Fe), du zinc (Zn), du cuivre (Cu), du manganèse (Mn), du sélénium (Se).

3. Solution hypertonique selon la revendication 1 ou 2, **caractérisée par le fait que** dans la solution ionique hyperosmotique à base d'eau de mer, la composition en les éléments autres que le sodium, le potassium, les chlorures, le calcium et le magnésium est qualitativement identique à celle de l'eau de mer.

4. Médicament **caractérisé par le fait qu'**il est à base d'une solution ionique hypertonique à base d'eau de mer non diluée et présentant
- une osmolalité comprise entre 500 et 700 mOsm/kg, de préférence entre 550 et 700 mOsm/kg, et plus préférentiellement encore entre 600 et 650 mOsm/kg,
- un pH compris entre 7,5 et 9,
- une composition du point de vue ionique qui est qualitativement celle de l'eau de mer, qui est telle que le rapport Na⁺/Mg⁺⁺ soit inférieur à 6, de préférence compris entre 3,5 et 5, et plus préférentiellement encore compris entre 4,5 et 5.

5. Médicament selon la revendication 4, **caractérisé par le fait que** les concentrations en Na⁺, K⁺, Mg⁺⁺, Ca⁺⁺ et Cl⁻ sont les suivantes :
- pour Na⁺, de 5000 à 19000, de préférence de 5500 à 8000, et plus préférentiellement encore de 6000 à 6500 mg/l
- pour K⁺, de 50 à 650, de préférence de 100 à 250 mg/l
- pour Mg⁺⁺, de 1000 à 4000, de préférence de 1000 à 2000 et plus preférentiellement encore de 1100 a 1500 mg/l
- pour Ca⁺⁺, de 300 à 650, de préférence de 350 à 450 mg/l
- pour Cl, de 8000 à 33000, de préférence de 9000 à 15000, et plus préférentiellement encore de 10000 à 13000 mg/l.

6. Médicament selon la revendication 4 ou la revendication 5, **caractérisé par le fait que** la solution ionique hyperosmotique à base d'eau de mer contient également du brome (Br), de l'aluminium (Al), du fluor (F), de l'iode (I), du fer (Fe), du zinc (Zn), du cuivre (Cu), du manganèse (Mn), du sélénium (Se).

7. Médicament selon l'une quelconque des revendications 4 à 6, **caractérisé par le fait que** dans la solution ionique hyperosmotique à base d'eau de mer, la composition en les éléments autres que le sodium, le potassium, les chlorures, le calcium et le magnésium est qualitativement identique à celle de l'eau de mer.

8. Médicament selon l'une quelconque des revendications 4 à 7, qui est sous la forme d'une solution pour pulvérisation nasale.

9. Médicament selon l'une quelconque des revendications 4 à 8, qui est dépourvu de tout agent conservateur ou stabilisant.

10. Utilisation de solution ionique hypertonique à base d'eau de mer selon l'une quelconque des revendications 1 à 3, pour la préparation d'un médicament destiné au traitement curatif ou prophylactique de la congestion nasale.

11. Utilisation selon la revendication 10, pour le traitement curatif ou prophylactique de la congestion nasale chez des patients traités par ou sensibles aux corticostéroïdes, en particulier aux corticostéroïdes pour administration par voie nasale.

12. Procédé de préparation de solution ionique selon l'une quelconque des revendications 1 à 3, selon lequel l'eau de mer est traitée par électrodialyse.

13. Procédé selon la revendication 12, **caractérisé par le fait que**, successivement :
- on prélève, en tant que matière première, de l'eau de mer d'une teneur en sels supérieure à 32g/l, avantageusement par une profondeur de 5 à 10 mètres dans une zone à forts mouvements de courant,
- on clarifie, filtre et analyse cette eau,
- si l'osmolalité de l'eau de mer clarifiée et filtrée est supérieure à l'osmolalité souhaitée, on la désode par électrodialyse jusqu'à l'obtention de l'osmolalité souhaitée qui est supérieure à 500 mOsm/kg,
- si l'osmolalité de l'eau de mer décantée est inférieure à l'osmolalité souhaitée, on la concentre jusqu'à l'obtention de l'osmolalité souhaitée qui est inférieure à 700 mOsm/kg,
- on ajuste les concentrations ioniques par électrodialyse sélective,
- on filtre et on stocke le produit dans des conditions stériles.

## Patentansprüche

1. Unverdünnte hypertonische Ionenlösung auf der Basis von Meerwasser, die Folgendes aufweist:
- eine Osmolalität zwischen 500 und 700 mOsm/kg, vorzugsweise zwischen 550 und 700 mOsm/kg und noch stärker bevorzugt zwischen 600 und 650 mOsm/kg
- einen pH-Wert zwischen 7,5 und 9,
- eine Zusammensetzung hinsichtlich der Ionen, die qualitativ diejenige von Meerwasser und derart ist, dass das Verhältnis Na⁺/Mg⁺⁺ kleiner als 6, vorzugsweise zwischen 3,5 und 5 und noch stärker bevorzugt zwischen 4,5 und 5 ist und deren Konzentrationen an Na⁺ K⁺, Mg⁺⁺, Ca⁺⁺ und Cl⁻ die Folgenden sind:
- für Na⁺ von 5000 bis 19000, vorzugsweise von 5500 bis 8000 und noch stärker bevorzugt von 6000 bis 6500 mg/l
- für K⁺ von 50 bis 650, vorzugsweise von 100 bis 250 mg/l
- für Mg⁺⁺ von 1000 bis 4000, vorzugsweise von 1000 bis 2000 und noch stärker bevorzugt von 1100 bis 1500 mg/l
- für Ca⁺⁺ von 300 bis 1200, vorzugsweise von 350 bis 450 mg/l
- für Cl⁻ von 8000 bis 33000, vorzugsweise von 9000 bis 15000 und stärker bevorzugt von 10000 bis 13000 mg/l.

2. Hypertonische Lösung nach Anspruch 1, **dadurch gekennzeichnet, dass** sie auch Brom (Br), Aluminium (Al), Fluor (F), Iod (I), Eisen (Fe), Zink (Zn), Kupfer (Cu), Mangan (Mn), Selen (Se) enthält.

3. Hypertonische Lösung nach Anspruch 1 oder 2, **dadurch gekennzeichnet, dass** in der hyperosmotischen Ionenlösung auf der Basis von Meerwasser die Zusammensetzung an anderen Elementen als Natrium, Kalium, Chloriden, Calcium und Magnesium qualitativ mit derjenigen von Meerwasser identisch ist.

4. Arzneimittel, **dadurch gekennzeichnet, dass** es auf einer unverdünnten hypertonischen Ionenlösung auf der Basis von Meerwasser basiert und Folgendes aufweist
- eine Osmolalität zwischen 500 und 700 mOsm/kg, vorzugsweise zwischen 550 und 700 mOsm/kg und noch stärker bevorzugt zwischen 600 und 650 mOsm/kg,
- einen pH-Wert zwischen 7,5 und 9,
- eine Zusammensetzung hinsichtlich der Ionen, die qualitativ diejenige von Meerwasser und derart ist, dass das Verhältnis Na⁺/Mg⁺⁺ kleiner als 6, vorzugsweise zwischen 3,5 und 5 und noch stärker bevorzugt zwischen 4,5 und 5 ist.

5. Arzneimittel nach Anspruch 4, **dadurch gekennzeichnet, dass** die Konzentrationen an Na⁺ K⁺, Mg⁺⁺, Ca⁺⁺ und Cl⁻ die Folgenden sind:
- für Na⁺ von 5000 bis 19000, vorzugsweise von 5500 bis 8000 und noch stärker bevorzugt von 6000 bis 6500 mg/l
- für K⁺ von 50 bis 650, vorzugsweise von 100 bis 250 mg/l
- für Mg⁺⁺ von 1000 bis 4000, vorzugsweise von 1000 bis 2000 und noch stärker bevorzugt von 1100 bis 1500 mg/l
- für Ca⁺⁺ von 300 bis 650, vorzugsweise von 350 bis 450 mg/l
- für Cl⁻ von 8000 bis 33000, vorzugsweise von 9000 bis 15000 und stärker bevorzugt von 10000 bis 13000 mg/l.

6. Arzneimittel nach Anspruch 4 oder Anspruch 5, **dadurch gekennzeichnet, dass** die hyperosmotische Ionenlösung auf der Basis von Meerwasser auch Brom (Br), Aluminium (Al), Fluor (F), Iod (I), Eisen (Fe), Zink (Zn), Kupfer (Cu), Mangan (Mn), Selen (Se) enthält.

7. Arzneimittel nach einem der Ansprüche 4 bis 6, **dadurch gekennzeichnet, dass** in der hyperosmotischen Ionenlösung auf der Basis Meerwasser die Zusammensetzung an anderen Elementen als Natrium, Kalium, Chloriden, Calcium und Magnesium qualitativ mit derjenigen von Meerwasser identisch ist.

8. Arzneimittel nach einem der Ansprüche 4 bis 7, das in Form einer Lösung als Nasenspray vorliegt.

9. Arzneimittel nach einem der Ansprüche 4 bis 8, das frei von jeglichem Konservierungs- oder Stabilisierungsmittel ist.

10. Verwendung der hypertonischen Ionenlösung auf der Basis von Meerwasser nach einem der Ansprüche 1 bis 3 zur Herstellung eines Arzneimittels für die kurative oder prophylaktische Behandlung einer verstopften Nase.

11. Verwendung nach Anspruch 10 für die kurative oder prophylaktische Behandlung einer verstopften Nase bei Patienten, die mit Kortikosteroiden behandelt werden oder empfindlich gegenüber Kortikosteroiden, insbesondere auf nasalem Weg verabreichten Kortikosteroiden, sind.

12. Verfahren zur Herstellung einer Ionenlösung nach einem der Ansprüche 1 bis 3, wobei das Meerwasser mittels Elektrodialyse behandelt wird.

13. Verfahren nach Anspruch 12, **dadurch gekennzeichnet, dass** man nacheinander:
- als Ausgangsstoff Meerwasser mit einem Gehalt an Salzen von mehr bis 32 g/l, vorzugsweise aus einer Tiefe von 5 bis 10 Metern in einer Zone mit starken Strömungsbewegungen, entnimmt,
- dieses Wasser klärt, filtriert und analysiert,
- wenn die Osmolalität des geklärten und filtrierten Meerwassers größer ist als die gewünschte Osmolalität, es durch Elektrodialyse entsalzt, bis die gewünschte Osmolalität, die über 500 mOsm/kg beträgt, erhalten wird,
- wenn die Osmolalität des dekantierten Meerwassers niedriger ist als die gewünschte Osmolalität, es aufkonzentriert, bis die gewünschte Osmolalität, die kleiner als 700 mOsm/kg ist, erhalten wird,
- die Ionenkonzentrationen durch selektive Elektrodialyse einstellt,
- filtriert und das Produkt unter sterilen Bedingungen aufbewahrt.

## Claims

1. Ionic hypertonic solution based on undiluted seawater, having:
- an osmolality of between 500 and 700 mOsm/kg, preferably between 550 and 700 mOsm/kg, and even more preferentially between 600 and 650 mOsm/kg,
- a pH of between 7.5 and 9,
- a composition from the ionic point of view which is qualitatively that of seawater and which is such that the Na⁺/Mg⁺⁺ ratio is less than 6, preferably between 3.5 and 5, and even more preferentially between 4.5 and 5 and the Na⁺, K⁺, Mg⁺⁺, Ca⁺⁺ and Cl⁻ concentrations of which are the following:
- for Na⁺, from 5000 to 19 000, preferably from 5500 to 8000, and even more preferentially from 6000 to 6500 mg/l
- for K⁺, from 50 to 650, preferably from 100 to 250 mg/l
- for Mg⁺⁺, from 1000 to 4000, preferably from 1000 to 2000, and even more preferentially from 1100 to 1500 mg/l
- for Ca⁺⁺, from 300 to 1200, preferably from 350 to 450 mg/l
- for Cl⁻, from 8000 to 33 000, preferably from 9000 to 15 000, and even more preferentially from 10 000 to 13 000 mg/l.

2. Hypertonic solution according to Claim 1, **characterized in that** it also contains bromine (Br), aluminium (Al), fluorine (F), iodine (I), iron (Fe), zinc (Zn), copper (Cu), manganese (Mn) and selenium (Se).

3. Hypertonic solution according to Claim 1 or 2, **characterized in that**, in the ionic hyperosmotic solution based on seawater, the composition of the elements other than sodium, potassium, chlorides, calcium and magnesium is qualitatively identical to that of seawater.

4. Medicament **characterized in that** it is based on an ionic hypertonic solution based on undiluted seawater and having
- an osmolality of between 500 and 700 mOsm/kg, preferably between 550 and 700 mOsm/kg, and even more preferentially between 600 and 650 mOsm/kg,
- a pH of between 7.5 and 9,
- a composition from the ionic point of view which is qualitatively that of seawater, which is such that the Na⁺/Mg⁺⁺ ratio is less than 6, preferably between 3.5 and 5, and even more preferentially between 4.5 and 5.

5. Medicament according to Claim 4, **characterized in that** the Na⁺, K⁺, Mg⁺⁺, Ca⁺⁺ and Cl⁻ concentrations are the following:
- for Na⁺, from 5000 to 19 000, preferably from 5500 to 8000, and even more preferentially from 6000 to 6500 mg/l
- for K⁺, from 50 to 650, preferably from 100 to 250 mg/l
- for Mg⁺⁺, from 1000 to 4000, preferably from 1000 to 2000 and even more preferentially from 1100 to 1500 mg/l
- for Ca⁺⁺, from 300 to 650, preferably from 350 to 450 mg/l
- for Cl⁻, from 8000 to 33 000, preferably from 9000 to 15 000, and even more preferentially from 10 000 to 13 000 mg/l.

6. Medicament according to Claim 4 or Claim 5, **characterized in that** the ionic hyperosmotic solution based on seawater also contains bromine (Br), aluminium (Al), fluorine (F), iodine (I), iron (Fe), zinc (Zn), copper (Cu), manganese (Mn) and selenium (Se).

7. Medicament according to any one of Claims 4 to 6, **characterized in that**, in the ionic hyperosmotic solution based on seawater, the composition of the elements other than sodium, potassium, chlorides, calcium and magnesium is qualitatively identical to that of seawater.

8. Medicament according to any one of Claims 4 to 7, which is in the form of a solution for nasal spray.

9. Medicament according to any one of Claims 4 to 8, which is free of any preservative or stabilizer.

10. Use of ionic hypertonic solution based on seawater according to any one of Claims 1 to 3, for preparing a medicament intended for the curative or prophylactic treatment of nasal congestion.

11. Use according to Claim 10, for the curative or prophylactic treatment of nasal congestion in patients treated with or sensitive to corticosteroids, in particular to corticosteroids for nasal administration.

12. Method for preparing ionic solution according to any one of Claims 1 to 3, according to which the seawater is treated by electrodialysis.

13. Method according to Claim 12, **characterized in that**, successively:
- seawater having a salt content greater than 32 g/l is taken, as starting material, advantageously from a depth of 5 to 10 metres in an area with strong currents,
- this water is clarified, filtered and analysed,
- if the osmolality of the clarified and filtered seawater is greater than the desired osmolality, sodium is removed by electrodialysis until the desired osmolality, which is greater than 500 mOsm/kg, is obtained,
- if the osmolality of the decanted seawater is less than the desired osmolality, it is concentrated until the desired osmolality, which is less than 700 mOsm/kg, is obtained,
- the ionic concentrations are adjusted by selective electrodialysis,
- the product is filtered and stored under sterile conditions.
